# EUROPEAN PATENT SPECIFICATION

(11) **EP 1 944 247 B1**
(45) Date of publication and mention of the grant of the patent: **29.02.2012**
(21) Application number: 06798321.3
(22) Date of filing: 25.09.2006
(51) Int. Cl.: B65D 85/16, A41C 3/04, B65D 33/00, B65D 77/28

(54) **BREAST MILK PAD PACKAGE**
STILLEINLAGEPACKUNG
EMBALLAGE DE TAMPON D'ALLAITEMENT

(30) Priority: 01.11.2005 JP 2005318430
(43) Date of publication of application: 16.07.2008
(73) Proprietor: Uni-Charm Corporation, Ehime 799-0111 (JP)
(72) Inventor: SAKAGUCHI, Satoru, Kanonji-shi Kagawa 769-1602 (JP); SUGA, Ayami, Kanonji-shi Kagawa 769-1602 (JP)
(74) Representative: Stark, Gordon Drummond
(86) International application number: PCT/JP2006/318996
(87) International publication number: WO 2007/052423

(56) References cited:
- DE-C1- 19 724 352
- JP-A- 9 137 307
- JP-A- 11 229 206
- JP-A- 2000 247 358
- JP-A- 2003 104 441
- US-A1- 2002 022 815
- US-A1- 2003 158 532

## Description

### TECHNICAL FIELD

The present invention relates to a disposable nursing pad wrapped body which can be easily worn again after breast-feeding, without losing the shape of the nursing pad when breast-feeding.

### BACKGROUND ART

In general, a large amount of a mother's milk is secreted in response to a sucking stimulation from a suckling baby. In some cases, the mother's milk is secreted without such a sucking stimulation. Also, the left and right breasts are linked in that when a mother gives one breast to a suckling baby, the mother's milk is also secreted from the other breast. In order to keep her clothing dry from such undesired secretion of a mother's milk, a nursing mother wears nursing pads in contact with the inner face of the user's underclothing such as a bra or the like such that they cover the breasts, thereby absorbing excess milk.

In general, a nursing mother, wearing a nursing pad on the inner face of a bra, displaces the bra downward and obliquely to expose her breast. At this stage, the nursing pad is sandwiched between the bra and the outer face of the lower portion of the breast, leading to application of pressure to the nursing pad. This often leads to the nursing pad losing its shape. A nursing pad which has lost its shape adheres to the inner face of the bra without recovery of shape by actions of an adhesive applied to the clothing used for preventing displacement of the nursing pad. This leads to difficulty in the user wearing the nursing pad again in a restored state.

On the other hand, Patent Document 1 discloses a nursing pad having a structure in which recessed grooves 110 are formed across the portion which is to be in contact with the user's nipple (see Fig. 7). With such a nursing pad disclosed in Patent Document 1, the nursing pad is manufactured without heat application. This provides a dome-shaped nursing pad corresponding to the shape of the user's breast while keeping the nursing pad feeling soft.

Patent Document 1: Japanese Unexamined Patent Application Publication No. 2000-178805.

US Patent Application Publication No. 2002/0022815 A1 discloses a nursing pad comprising an inner, flexible, water permeable layer, an exterior, flexible, water impermeable layer having a peripheral portion bonded to the inner layer, an absorbent layer interposed between the inner and exterior layers and a slit extending from a periphery of the pad inward to form separated portions on both sides thereof so that the pad can form to the contour of the user's breast. The separated portions are overlapped and an adhesive is provided on at least one of the separated portions for maintaining the overlap of the separated portions.

US Patent Application Publication No. 2003/0158532 A1 discloses a disposable absorbent article to be worn about the lower torso of a wearer. The absorbent article comprises a top sheet, back sheet and a core disposed therebetween and includes at least one serviceable indicium that facilitates an easy, intuitive change by aligning the article relative to an anatomical feature of the wearer or a component of the article.

German Patent No.19724352 C1 discloses a brassiere insert for a nursing mother for protecting the breast and/or clothing by an absorbing liquid. The insert consists of one or more woven material layers formed as individual flat layer pieces which are sewn together along a seam. The seam is formed by a shaping seam which extends across and above the insert and which provides the insert with a preformed shape at least approximately matching the breast to be protected.

Japanese Patent Publication No. 11229206 A discloses a pad for a brassiere provided by forming a bending part extending in the vertical direction of the pad at approximately the middle part of the pad in longitudinal direction. The pad serves to retain the shape of the brassiere.

Japanese Patent Publication No. 9137307 A discloses a pad for a breast having an upper side consisting of a long curved line containing a covering part for covering a papilla, a short curved line, a connecting part of both lines forming a cut in part and a lower end which is a curved line along a lower line of the outside shape of a breast. An almost triangular indented part consisting of three lines for indenting is formed by taking the covering part as its top and having a prescribed angle. The indented part has a thinner thickness than that of the pad parts in both sides and is freely bendable along the indenting lines.

### DISCLOSURE OF THE INVENTION

### Problems to be Solved by the Invention

As described above, the nursing pad disclosed in Patent Document 1 has a structure in which the recessed grooves 110 are formed on both sides across the portion which is to be in contact with the user's nipple. However, the portions where the recessed grooves 110 are formed differ from the portions along which the nursing pad often twists at the time of breast-feeding. Accordingly, the nursing pad easily twists excessively with each of the recessed grooves 110 as a folding axis, leading to the nursing pad losing its shape. This leads to difficulty in the user wearing the nursing pad again.

The present invention has been made in view of the aforementioned problems. Accordingly, it is an object thereof to provide a disposable nursing pad which can be worn again after breast-feeding, and which does not lose its shape during breast-feeding.

### Means for Solving the Problems

The present inventors have diligently studied these problems, giving attention to the fact that the nursing pad often twists obliquely when a nursing mother displaces the bra downward and obliquely such that she can expose or cover her breast. As a result, the present inventors have made an arrangement in which a nursing pad main body includes a first folding portion, which serves as a folding axis that allows the nursing pad main body to be folded, provided such that it passes near an apex of the aforementioned nursing pad, and extends up to around the perimeter thereof, with a display being provided on a wrapping member or in an instruction manual, providing information prompting the user to wear the nursing pad main body with the first folding portion being positioned obliquely from the upper-outer side to the lower-inner side of the user's breast, whereby the- present invention was realized. More specifically, the present invention provides the following arrangements.

In a first aspect of the present invention, a nursing pad wrapped body including: a nursing pad, the nursing pad having a pair of nursing pad main bodies formed in a shape that allows a user's breasts to be covered; and a wrapping member, the wrapping member wraps the nursing pad, each nursing pad main body being formed in the shape of a dome having a curved face with an apex provided at a top end thereof, each nursing pad main body including: a liquid-impermeable sheet provided on an outer face of the nursing pad main body; a liquid-permeable sheet, provided on the breast side of the liquid-impermeable sheet and joined to the liquid-impermeable sheet at the periphery thereof; an absorber, provided between the liquid-impermeable sheet and the liquid-permeable sheet, which absorbs and retains a mother's milk; and a first folding portion, arranged such that it passes near the apex of the nursing pad main body and extends to around the perimeter of the nursing pad main body, which serves as a folding axis along which the nursing pad main body can be folded; wherein the liquid-impermeable sheet has a bending responsive means positioned across the first folding portion, wherein the bending responsive means is an elastic member. Also disclosed herein is a display region, providing visual information at a position at which the information can be confirmed from outside of the wrapping member, which prompts the user to wear the nursing pad main body with the first folding portion being positioned obliquely from the upper-outer side to the lower-inner side of the user's breast.

The nursing pad wrapped body described in the first aspect includes the nursing pad main body having the first folding portion, which serves as a folding axis along which the nursing pad main body can be folded, provided such that the folding portion passes through near the apex of the aforementioned nursing pad main body, and extends up to around the perimeter thereof. Also disclosed herein is a display region on the wrapping member which provides the information that instructs the user to wear the nursing pad main body with the first folding portion being positioned obliquely from the upper-outer side to the lower-inner side of the user's breast. With such an arrangement, the user wears the nursing pad main body according to a wearing method displayed on the wrapping member. Furthermore, such an arrangement allows the nursing pad main body to be folded following the shape of the user's breast with the first folding portion as a folding axis when a nursing mother displaces the bra downward and obliquely before breast-feeding. That is to say, the nursing pad main body is folded along the first folding portion, thereby effectively preventing the nursing pad from losing its shape. This allows the user to wear the nursing pad main body again along with the bra in the normal state as before. Furthermore, such an arrangement prevents a mother's milk stored in the absorber from escaping due to the absorber being twisted.

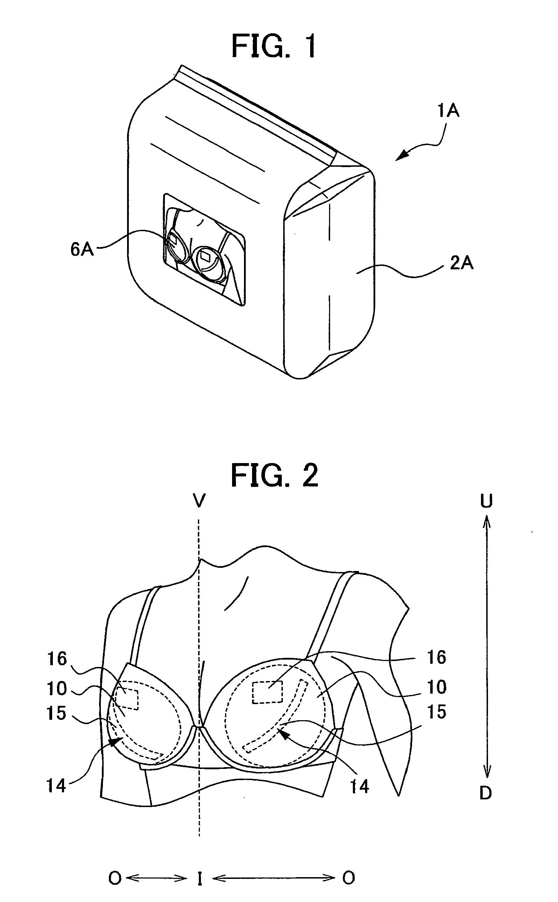

In the present specification, the phrase "pass through the user's body obliquely" as used here means that the folding portion passes through the user's body obliquely with respect to the longitudinal axis passing through the center of the user's body when the user is being in the standing position. The term "upper portion" or "upper side" as used here represents the direction of the user's head along the longitudinal direction. On the other hand, the term "lower portion" or "lower side" as used here represents the direction of the user's foot along the longitudinal direction. The term "outer side" as used here represents the side of the user's arm with respect to the user's body. On the other hand, the term "inner side" as used here represents the side of the longitudinal axis that passes through the center of the user's body having the breasts. As shown in Fig. 2, the "center axis" is indicated by the line V, and the "upper portion" and "upper side" are indicated by the reference numeral "U". Also, the "lower portion" and "lower side" are indicated by the reference numeral "D". The "outer side" is indicated by the reference numeral "O", and the "inner side" indicated denoted by reference numeral "I".

In the present specification, the term "apex" as used here represents the top end of the curved face of the nursing pad formed in the shape of a dome, i.e., when a perimeter of the nursing pad is in contact with a surface, a point which is farthest from this surface. On the other hand, the term "perimeter" as used here represents the portion near the outer edge of the nursing pad main body.

Also disclosed herein is an instruction manual illustrating a usage method of the nursing pad wherein the wrapping member wraps the nursing pad and the instruction manual, and wherein the instruction manual displays contents in which the nursing pad main body is worn with the first folding portion being positioned obliquely from the upper-outer side to the lower-inner side of the user's breast.

In a second aspect of the nursing pad as described in the first aspect of the present invention, the nursing pad main body, in a state folded into two with the first folding portion as a folding axis, is wrapped by the wrapping member. Also disclosed herein is a nursing pad in which the display region displays a state in which the nursing pad main body is worn such that the first folding portion is positioned obliquely from the upper-outer side to the lower-inner side of the user's breast.

In a third aspect of the nursing pad as described in the first aspect of the present invention, in which the nursing pad main body further includes fastening means for fastening the nursing pad main body to the user's clothing at a region other than the first folding portion and a region orthogonal to the first folding portion. Also disclosed herein is a nursing pad in which the display region provides information with respect to a method for fastening the nursing pad main body to the user's clothing using the fastening means with the first folding portion being positioned obliquely from the upper-outer side to the lower inner side of the user's breast.

Also disclosed herein is an instruction manual including a display of an example such that the first folding portion is positioned obliquely from the upper-outer side to the lower-inner side of the user's breast.

In a fourth aspect of the nursing pad as described in the first aspect of the present invention, in which there is a difference in stiffness between the first folding portion and a region other than the first folding portion of the nursing pad main body.

### Effects of the Invention

The present invention provides a disposable nursing pad which allows the user to easily wear it again while preventing the nursing pad from losing its shape during the breast-feeding.

### BRIEF DESCRIPTION OF THE DRAWINGS

Fig. 1 is a perspective view which shows a nursing pad wrapped body according to a first embodiment;
Fig. 2 is an enlarged view which shows a display region provided to the nursing pad wrapped body according to the first embodiment;
Fig. 3 is a plan view of a nursing pad main body as viewed from the outside (from the side of the clothing);
Fig. 4 is a cross-sectional view taken along line X-X' in Fig. 3;
Fig. 5 is a perspective view of the nursing pad main body;
Fig. 6 is a plan view which shows a nursing pad wrapped body according to a second embodiment;

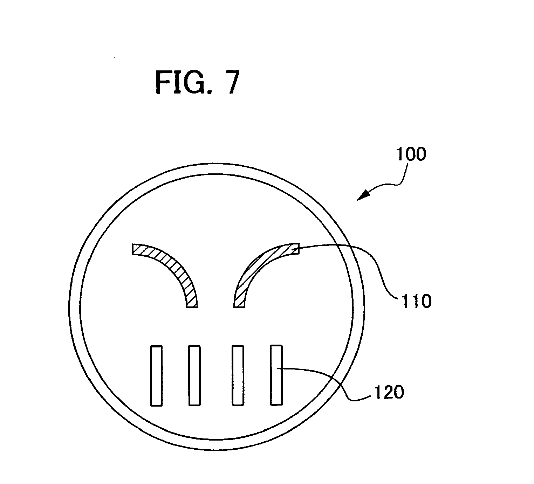

Fig. 7 is a plan view of a conventional nursing pad main body as viewed from the outside (from the side of the clothing);
Fig. 8 is a perspective view of an example of a nursing pad main body; and
Fig. 9 is a cross-sectional view taken along line X-X' in Fig. 8.

### PREFERRED MODE FOR CARRYING OUT THE INVENTION

A description will be provided regarding embodiments according to the present invention with reference to the drawings.

### First Embodiment

### [Overall Structure of Nursing Pad Wrapped Body]

Fig. 1 is a perspective view of a nursing pad wrapped body 1A according to a first embodiment. The nursing pad wrapped body 1A is a pack which packs multiple nursing pads including pairs of nursing pad main bodies, wrapped in a wrapping member 2A. The nursing pad wrapped body 1A includes a display region 6A for displaying a method of wearing a nursing pad. Fig. 2 is an enlarged diagram which shows the display region 6A.

### [Wrapping member]

The wrapping member 2A is formed by a known and conventional manufacturing method using a known and conventional material. Examples of the materials preferably employed include a polyethylene film, etc. Examples of modifications of the present embodiment include a box-type nursing pad wrapped body. In this case, examples of the materials of the wrapped body include cardboard, etc.

### [Nursing Pad]

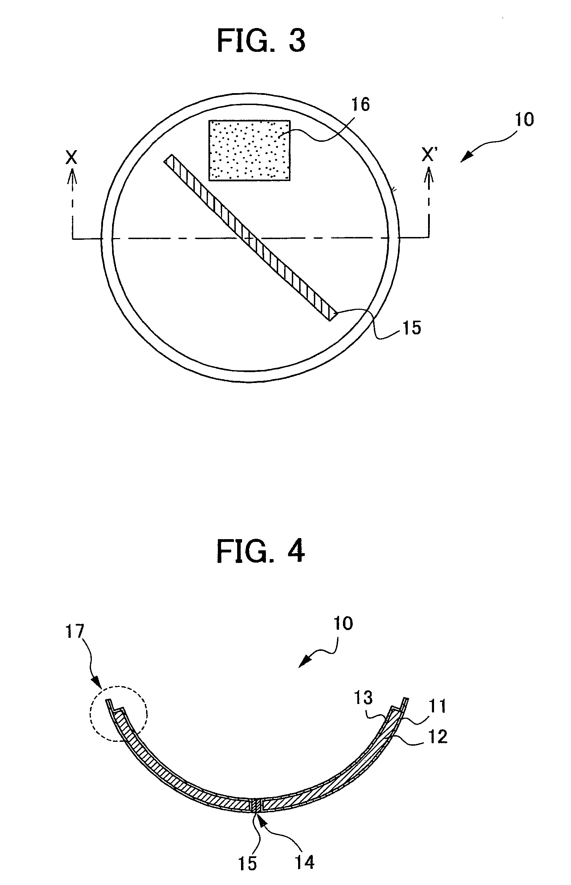

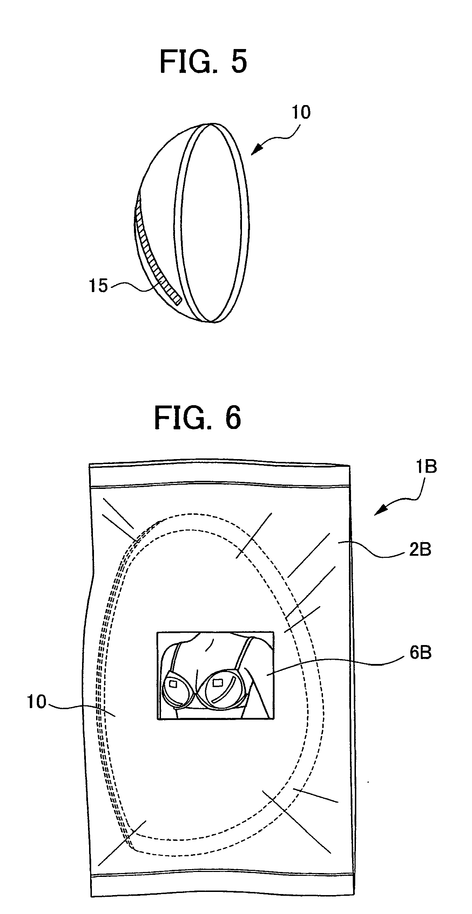

Fig. 3 is a plan view which shows the nursing pad main body 10 for the right breast included in the nursing pad wrapped body 1A according to the present invention (For the sake of simplification, descriptions will be made regarding only the nursing pad main body 10 for the right breast). Fig. 4 is a cross-sectional view taken along line X-X' in Fig. 3. Fig. 5 is a perspective view of the nursing pad main body 10. As shown in these drawings, the nursing pad main body 10 is formed in the shape of a dome.

### Nursing Pad Main Body

The nursing pad main body 10 includes: a liquid-impermeable sheet 11 which forms the external shape; a liquid-permeable sheet 13 having liquid permeability provided on the breast side of the liquid-impermeable sheet 11 with the perimeter 17 thereof being adhered to the perimeter 17 of the liquid-impermeable sheet 11; and an absorber 12, which is provided between the liquid-impermeable sheet 11 and the liquid-permeable sheet 13, and has a function of absorbing and holding a mother's milk. The nursing pad main body 10 has a first folding portion 15 which serves as a folding axis along which the nursing pad main body 10 can be folded. The first folding portion 15 is formed such that it passes near the apex 14 of the nursing pad main body 10, and extends around the perimeter portion 17 of the nursing pad. Furthermore, fastening means 16 are provided to a part of the liquid-impermeable sheet 11, except for the first folding portion 15 and the linear portion orthogonal to the first folding portion 15. The fastening means 16 allow the nursing pad main body 10 to be fastened to the user's clothing.

### Liquid-Permeable Sheet

The liquid-permeable sheet 13 is provided on the breast side of the dome-shaped nursing pad main body 10. Accordingly, the liquid-permeable sheet 13 is directly in contact with the user's breast, and is formed of a material which feels soft. Furthermore, the liquid-permeable sheet 13 needs to receive the mother's milk, and to introduce the mother's milk to the absorber 12 provided on the outside thereof. Accordingly, the liquid-permeable sheet 13 is formed of a liquid-permeable material. Specifically, the liquid-permeable sheet 13 is formed of hydrophilic fibrous non-woven fabric. Alternatively, the liquid-permeable sheet 13 is formed of hydrophobic fibrous non-woven fabric or film having a great number of pores.

### Liquid-Impermeable Sheet

The liquid-impermeable sheet 11 is provided on the outer face of the dome-shaped nursing pad main body 10, and forms the external shape of the nursing pad main body 10. The liquid-impermeable sheet 11 is formed of a liquid-impermeable material such that it protects the user's clothing from being damp due to an undesired escape of the mother's milk held by the absorber layer 12. Specific examples of such materials include: a moisture-permeable and liquid-impermeable drawn plastic film containing an inorganic fine particles such as silica, alumina, or the like; a laminate of a liquid-impermeable film and a fibrous non-woven fabric; a laminate of a moisture-permeable and liquid-impermeable film and a fibrous non-woven fabric; etc. Also, liquid-impermeable sheet 11 may be formed of a composite non-woven fabric having a laminate structure of spunbonded non-woven fabric and a meltblown non-woven fabric.

### Absorber Layer

The absorber 12 is formed of a mixture of particulate or fibrous high water absorption polymer and fluff pulp, or a mixture of particulate or fibrous high water absorption polymer, fluff pulp, and thermoplastic synthetic resin fiber. More specifically, such a mixture is pressed into a sheet with a predetermined thickness, thereby forming the absorber 12. The absorber 12 is formed with a predetermined thickness over the entire area by pressing processing. Thus, the absorber 12 exhibits a higher stiffness than that of the liquid permeable sheet 13 and the liquid impermeable sheet 11 described above. Note that the entire area of the absorber 12 is preferably wrapped in tissue paper in order to prevent the absorber 12 losing its shape, and to prevent polymer from escaping from the absorber.

The absorber 12 adheres to between the inner face of the liquid permeable sheet 13 or the liquid impermeable sheet 11 through the tissue paper using a hot melt adhesive or the like. Note that an arrangement may be made employing an SM non-woven fabric or an SMS non-woven fabric, instead of the tissue paper. Here, each of the SM non-woven fabric and the SMS non-woven fabric is a composite non-woven fabric formed in the shape of a sheet having a laminate structure in which a fibrous non-woven fabric, which is formed with a high strength and high flexibility using a spun-bonding method, is provided to at least one face of a fibrous non-woven fabric which is formed with a high fiber density using a meltblown method. With such an arrangement employing such a composite non-woven fabric, the fibers which form the fabric exhibit hydrophobicity. Accordingly, the absorber is preferably subjected to hydrophilic processing in order to improve the liquid-permeability. Note that examples of high water absorption polymers employed include: starch polymer; cellulose polymer; synthetic polymer; etc.

The adhesive is applied to the liquid-impermeable sheet 11, the liquid-permeable sheet 13, and the absorber 12, in any one of a spiral manner, an undulating manner, a zigzag manner, a dotted manner, and a stripe manner. With the present embodiment, the adhesive is applied to such a component sheet in such an application manner. Accordingly, the liquid-impermeable sheet 11 and the liquid-permeable sheet 13 are adhered to each other in a discontinuous manner, thereby causing the absorber 12 to adhere to the liquid-impermeable sheet 11 and the liquid-permeable sheet 13 in a discontinuous manner. As an adhesive, a hot melt adhesive or the like is employed. Either of an olefin adhesive or a styrene rubber adhesive can be employed as such an adhesive.

The fibrous non-woven fabric which can be employed in the present embodiment may be manufactured using any one of a spun-lace method, a needle punch method, a meltblown method, a thermal-bond method, a spun-bonding method, and a chemical-bonding method. The hydrophilic fibrous non-woven fabric can be formed of any one fiber selected from among a synthetic fiber, semi-synthetic fiber, and a regenerated fiber, which have been subjected to hydrophilic processing, or a composite fiber formed of a mixture of these fibers. The hydrophobic fibrous non-woven fabric is obtained from the synthetic fiber. The hydrophobic fibrous non-woven fabric may contain semi-synthetic fiber and regenerated fiber, which have been subjected to water-repellent processing. The synthetic fiber which can be employed is not restricted in particular. Specific examples of the synthetic fibers which can be employed include: a polyester fiber; a polyacrylonitrile fiber; a polyvinyl chloride fiber; a polyethylene fiber; a polypropylene fiber; and a polystyrene. Examples of the synthetic fibers which can be employed include: a sheath-core type composite fiber; a parallel-type composite fiber; a non-circular hollow fiber; microporous fiber; junction composite fiber; etc.

### First Folding Portion

The first folding portion 15 is provided to the nursing pad main body 10 such that it passes near the apex of the nursing pad main body 10, and continuously extends to around the perimeter portion 17. With such an arrangement, the absorber 12 is formed with different properties such as thickness, basic weight, or the like, at the first folding portion 15 as compared with the surroundings thereof, thereby providing difference in stiffness between the first folding portion 15 and the surroundings thereof. Also, the first folding portion 15 may be formed by embossing a part of the nursing pad main body 10. Such an arrangement also provides a difference in stiffness between the first folding portion 15 and the perimeter 17.

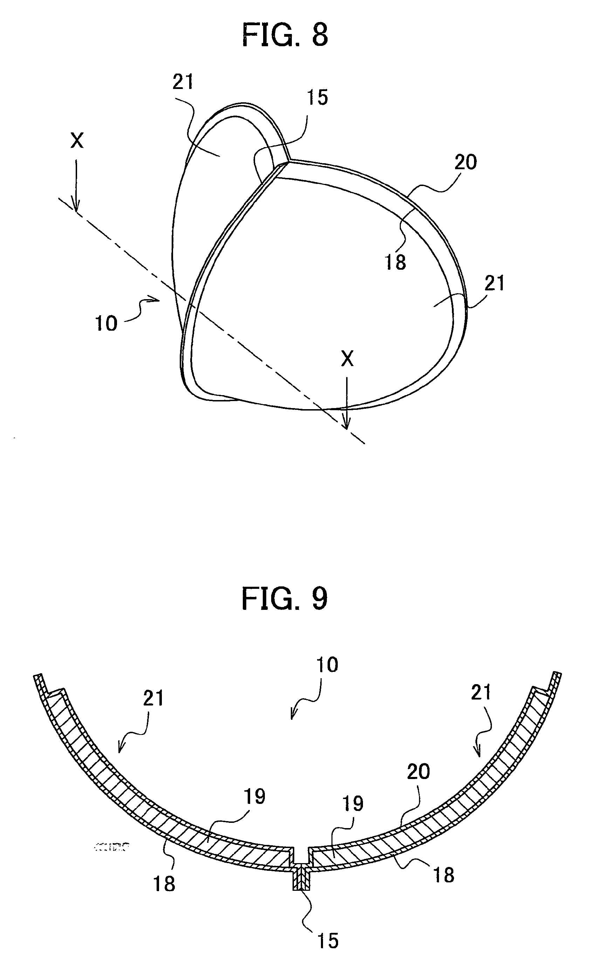

Also, an arrangement may be made in which the absorber 12 is not provided to a portion that passes near the apex of the nursing pad main body 10 and extends up to around the perimeter 17, and the liquid-permeable sheet 13 and the liquid-impermeable sheet 11 directly adhere to each other using a hot melt adhesive, thereby forming the first folding portion 15. Such an arrangement also provides difference in stiffness between the first folding portion and the surroundings thereof. Fig. 8 shows the perspective view, and Fig. 9 shows the cross sectional view taken along line X-X in Fig. 8 of the nursing pad main body 10 when the first folding portion 15 is provided by bonding. For example, an absorber 12 is separated into two pieces 19, a liquid-impermeable 11 sheet is separated into two pieces 18, and a liquid-permeable 13 sheet is separated into two pieces 20, and then the piece of absorber 19 is sandwiched between liquid-impermeable sheet 18 and the piece of the liquid-permeable sheet 20 respectively, thereby forming two pieces of nursing pad main body 21. With bonding each piece of nursing pad main body 21, the nursing pad main body 10 whose bonding line serves as the first folding portion 15 is formed.

Examples of the modifications of the nursing pad main body 10 according to the present embodiment include a modification in which the first folding portion is provided not in a continuous manner, but in a discontinuous manner. Specifically, the first folding portions are provided at a predetermined pitch giving consideration to the balance between the flexibility, which allows the nursing pad main body to be folded, and the water absorption capacity. With such a modification, the absorber 12 is not divided by the first folding portion 25. Thus, mother's milk is absorbed by the entire area of the absorber, thereby providing superior water absorption capacity.

Also, an arrangement may be made in which the first folding portion is provided in a discontinuous manner to regions other than the portion with which the user's nipple is in contact when the nursing pad main body is being worn. Specifically, the first folding portion is each provided to a part extending such that the folding portion passes near the apex of the nursing pad main body up to the portion around the perimeter, except for the portion which is to be in contact with the user's nipple, i.e., is each provided to separate regions on both sides across the central region. Here, each first folding portion is provided continuously. Also, each of the folding portions may be provided to both the regions in a discontinuous manner, instead of the aforementioned arrangement in which each first folding portion is provided continuously. Also, an arrangement may be made in which a single first folding portion is provided to only one of both of the sides across the central region. With such an arrangement, the first folding portion is not provided to the portion which is to be in contact with the user's nipple. This suppresses feelings of discomfort when the user is wearing the nursing pad main body.

Also, the nursing pad main body may further include a second folding portion provided such that it passes near the apex of the nursing pad main body, extends to around the perimeter of the nursing pad main body, in addition to the first folding portion. Here, the first folding portion and the second folding portion are formed orthogonal to each other. The second folding portion 57 is formed in the same way as the first folding portion 55 as described above. Such an arrangement allows the nursing pad main body to be easily folded not only along the first folding portion which serves as a folding axis, but also along the second folding portion which serves as another folding axis. This more effectively prevents the nursing pad main body from losing its shape, thereby allowing the user to easily wear it again.

The liquid-impermeable sheet has a bending responsive means positioned across the first folding portion. The bending-responsive means is an elastic member provided across the first folding portion. A panel formed in the shape of a sheet having elasticity is employed as such an elastic member. For example, a sheet formed of a foam material and a non-woven fabric such as a spunbonded non-woven fabric, through-air non-woven fabric, SMS non-woven fabric, etc., formed with a basic weight of 30 g/m² or more, exhibits increased elasticity. Accordingly, such sheets are employed as the elastic member having a function of satisfactory bending-responsive means, thus the shape of the nursing pad restores.

### [Fastening Means]

As described in the present embodiment, the nursing pad main body preferably includes the fastening means 16 which allows the nursing pad main body to be fastened to the user's clothing. Let us consider an arrangement in which the fastening means 16 is provided on the first folding portion 15. Such an arrangement leads to a problem of preventing the nursing pad main body 10 from being folded along the first folding portion 15 which serves as a folding axis. Accordingly, the fastening means 16 is preferably provided to a predetermined region in the nursing pad main body except for the first folding portion 15. Specific examples of fastening means 16 include: a means in which styrene rubber adhesive is applied; and a means in which a male mechanical fastener having multiple hooks is provided. With such an arrangement, the nursing pad main body 10 follows the bra by way of the fastening means 16. This effectively prevents the nursing pad main body 10 from losing its shape, thereby allowing the user to easily wear the nursing pad main body 10 again together with the bra in the normal state as before.

### [Display Region]

The display region 6A is provided to the wrapping member 2A by a known and conventional method such as a printing method or the like such that it provides visual confirmation from the outside of the wrapping member 2A. The contents of the display region are not restricted in particular, as long as the display region gives the user instructions that the nursing pad main body 10 should be worn with a first folding portion 15 being positioned obliquely from the upper-outer side to the lower-inner side of the user's breast. For example, the display region may be provided in a form of drawings or texts, which gives the user information with respect to the wearing state in which the user is wearing the nursing pad main body 10 in a proper manner, as shown in Fig. 2. Alternatively, the display region may be provided in a form which gives the user information with respect to the upper side and the lower side of the nursing pad main body 10, thereby guiding the user to a proper wearing state.

Note that another display region (not shown) may be directly provided to the nursing pad main body 10 such that it provides the same visual information as that of the display region 6A, which can be confirmed from the outside of the nursing pad main body 10. Specifically, the display region may be directly provided to the outer side of the surface of the liquid-impermeable sheet 11. Also, the display region may be directly provided to the inner side of the surface of the liquid-permeable sheet 13. Also, an arrangement may be made in which the display region is provided to the inner side or the outer side of the surface of an absorber 12, and the liquid-permeable sheet 13 or the liquid-impermeable sheet 11 has translucency, thereby allowing the user to see the display region through the translucent liquid-permeable sheet 13 or the translucent liquid-impermeable sheet 11. Also, the display region may be provided to the edge of the liquid-permeable sheet 13 or the liquid-impermeable sheet 11 in the form of a tag.

### [Instruction Manual]

Examples of the modifications of the present embodiment include an arrangement in which the nursing pad wrapped body 1A includes an instruction manual that provides the same visual information as that of the display region 6A, instead of or in addition to the display region 6A.

### [Operation and Effects]

A description will be given regarding the operation and effects of the nursing pad wrapped body 1A according to the present embodiment. The nursing pad wrapped body 1A according to the present embodiment has the display region 6A which guides the user to a proper wearing state in which the nursing pad main body 10 is being worn with the first folding portion 15 being positioned obliquely from the upper-outer side to the lower-inner side of the user's breast (with the above-described modification, the nursing pad wrapped body 1A includes an instruction manual that provides the same visual information). Such an arrangement allows the user to easily and properly wear the nursing pad main body 10 according to the instructions of the display region. Furthermore, the nursing pad main body 10 is easily folded along the first folding portion 15 that serves as a folding axis, in a case that the user displaces the nursing pad main body 10 along with the bra downward and obliquely when the user gives one breast to a suckling baby. Such an arrangement prevents the nursing pad main body 10 within the bra from folding in an irregular form, thereby effectively preventing the nursing pad main body 10 from losing its shape. Furthermore, in a case that the user wears the nursing pad main body 10 again together with the bra after breast-feeding, the nursing pad main body 10 can be easily restored to the normal state, thereby allowing the user to easily wear it again.

### Second Embodiment

A nursing pad wrapped body 1B according to a second embodiment is an individually-wrapped nursing pad wrapped body including the single nursing pad main body 10 wrapped in a wrapping member 2B. More specifically, the nursing pad wrapped body 1B is an individually-wrapped nursing pad wrapped body including the single nursing pad main body 10, folded into two with the first folding portion 15 as a folding axis, in a wrapped form. Fig. 6 is a plan view which shows the nursing pad wrapped body 1B. As shown in Fig. 6, the wrapping member 2B includes a display region 6B that provides the visual information with regard to a method of wearing the nursing pad which is a wrapped article included therein, which can be confirmed from the outside of the wrapping member 2B. The display region 6B provides the same displayed contents as those of the display region 6A according to the first embodiment. That is to say, Fig. 2 is also an enlarged view which shows the display region 6B.

### [Wrapping Member 2B]

The wrapping member 2B, which is a component of the nursing pad wrapped body 1B according to the present embodiment, is formed of a known and conventional material. On the other hand, the nursing pad wrapped body 1B is formed by a known and conventional manufacturing method. Specific examples of the materials preferably employed include a polyethylene film, etc.

### [Operation and Effects]

Description will be made regarding the operation and effects of the nursing pad wrapped body 1B according to the present embodiment. The nursing pad wrapped body 1B according to the present embodiment has the display region 6B which guides the user to a proper wearing state in which the nursing pad main body 10 is being worn with the first folding portion 15 being positioned obliquely from the upper-outer side to the lower-inner side of the user's breast. Such an arrangement allows the user to easily and properly wear the nursing pad main body 10 according to the instructions of the display region. Thus, the nursing pad wrapped body 1B according to the present embodiment provides the same operation and effects as those of the nursing pad wrapped body 1A according to the first embodiment. In addition, the nursing pad wrapped body 1B according to the present embodiment includes the display region 6B which displays the wearing method. Thus, in particular, the present embodiment is effectively applied to a portable nursing pad. While preferred embodiments of the present invention have been described and illustrated above, it is to be understood that they are exemplary of the invention and are not to be considered to be limiting.

## Claims

1. A nursing pad wrapped body (1A, 1 B) comprising:
a nursing pad having a pair of nursing pad main bodies (10) formed in a shape that allows a user's breasts to be covered; and
a wrapping member (2A, 2B) which wraps the nursing pad,
each nursing pad main body (10) being formed in the shape of a dome having a curved face with an apex provided at a top end thereof, each nursing pad main body (10) comprising:
a liquid-impermeable sheet (11) provided on an outer face of the nursing pad main body (10);
a liquid-permeable sheet (13), provided on the breast side of said liquid-impermeable sheet (11) and joined to said liquid-impermeable sheet (11) at the periphery thereof;
an absorber (12), provided between said liquid-impermeable sheet (11) and said liquid-permeable sheet (13), which absorbs and retains a mother's breast milk;
**characterised in that**
each nursing pad main body (10) comprises a first folding portion (15), arranged such that it passes near the apex of said nursing pad main body (10) and extends to around a perimeter (17) of said nursing pad main body (10), which serves as a folding axis along which said nursing pad main body (10) can be folded; and
the liquid-impermeable sheet (11) has a bending responsive means positioned across the first folding portion (15), wherein the bending responsive means is an elastic member.

2. The nursing pad wrapped body (1A, 1 B) according to Claim 1,
wherein said nursing pad main body (10), in a state folded into two with said first folding portion (15) as the folding axis, is wrapped by the wrapping member (2A, 2B).

3. The nursing pad wrapped body (1A, 1 B) according to Claim 1,
wherein said nursing pad main body (10) further includes a fastening means (16) for fastening said nursing pad main body (10) to the user's clothing at a region other than said first folding portion (15) and a region orthogonal to said first folding portion (15).

4. The nursing pad wrapped body (1A, 1 B) according to any one of Claims 1 to 3, wherein the absorber (12) is formed with different properties in thickness or basic weight at the first folding portion (15) as compared with the surroundings thereof, thereby providing a difference in stiffness between the first folding portion (15) and the surroundings thereof, or the first folding portion (15) is formed by embossing a part of the nursing pad main body (10), such that a difference in stiffness is provided between said first folding portion (15) and the perimeter (17).

5. The nursing pad wrapped body (1A, 1 B) according to any one of Claims 1 to 4, wherein the elastic member is a sheet formed of a foam material and a non-woven fabric formed with a basic weight of 30 g/m² or more.

6. The nursing pad wrapped body (1A, 1 B) according to any one of Claims 1 to 5, wherein the first folding portion (15) is provided discontinuously at a predetermined pitch.

7. The nursing pad wrapped body (1A, 1 B) according to any one of Claims 1 to 6, wherein the first folding portion (15) is provided in a discontinuous manner to separate regions on both sides across a central region of the nursing pad main body (10) and is not provided to the portion of the nursing pad main body (10) which is in contact with the user's nipple when the nursing pad main body (10) is being worn.

8. The nursing pad wrapped body (1A, 1 B) according to any one of Claims 1 to 6, wherein the first folding portion (15) is provided to only one side across a central region of the nursing pad main body (10) and is not provided to the portion of the nursing pad main body (10) which is in contact with the user's nipple when the nursing pad main body (10) is being worn.

9. The nursing pad wrapped body (1A, 1 B) according to any one of Claims 1 to 8, further comprising a second folding portion provided such that it passes near the apex of the nursing pad main body (10) and extends to the perimeter (17) thereof, the second folding portion being provided orthogonal to the first folding portion (15).

## Patentansprüche

1. Ein Stilleinlagenhüllkörper (1A, 1 B), der Folgendes beinhaltet:
eine Stilleinlage, die ein Paar Stilleinlagenhauptkörper (10) aufweist, die in einer Form gebildet sind, die das Bedecken der Brüste einer Benutzerin ermöglicht; und
ein Hüllungselement (2A, 2B), das die Stilleinlage umhüllt,
wobei jeder Stilleinlagenhauptkörper (10) in der Form einer Kuppel, die eine gekrümmte Fläche mit einem an einem oberen Ende davon bereitgestellten Scheitel aufweist, gebildet ist, wobei jeder Stilleinlagenhauptkörper (10) Folgendes beinhaltet:
eine flüssigkeitsundurchlässige Schicht (11), die auf einer äußeren Fläche des Stilleinlagenhauptkörpers (10) bereitgestellt ist;
eine flüssigkeitsdurchlässige Schicht (13), die auf der Brustseite der flüssigkeitsundurchlässigen Schicht (11) bereitgestellt und mit der flüssigkeitsundurchlässigen Schicht (11) an deren Peripherie verbunden ist;
einen Absorber (12), der zwischen der flüssigkeitsundurchlässigen Schicht (11) und der flüssigkeitsdurchlässigen Schicht (13) bereitgestellt ist, der die Muttermilch aus der Brust absorbiert und festhält;
**dadurch gekennzeichnet, dass**
jeder Stilleinlagenhauptkörper (10) einen ersten Faltabschnitt (15) beinhaltet, der so angeordnet ist, dass er nahe dem Scheitel des Stilleinlagenhauptkörpers (10) verläuft und sich bis ungefähr zu einem Umfang (17) des Stilleinlagenhauptkörpers (10) erstreckt, und der als eine Faltachse dient, entlang der der Stilleinlagenhauptkörper (10) gefaltet werden kann; und
die flüssigkeitsundurchlässige Schicht (11) ein auf Biegung reagierendes Mittel aufweist, das über den ersten Faltabschnitt (15) hinweg positioniert ist, wobei das auf Biegung reagierende Mittel ein elastisches Element ist.

2. Stilleinlagenhüllkörper (1A, 1 B) gemäß Anspruch 1,
wobei der Stilleinlagenhauptkörper (10), in einem zur Hälfte gefalteten Zustand mit dem ersten Faltabschnitt (15) als Faltachse, von dem Hüllungselement (2A, 2B) umhüllt ist.

3. Stilleinlagenhüllkörper (1A, 1 B) gemäß Anspruch 1,
wobei der Stilleinlagenhauptkörper (10) ferner ein Befestigungsmittel (16) zum Befestigen des Stilleinlagenhauptkörpers (10) an der Kleidung der Benutzerin in einem anderen Bereich als dem ersten Faltabschnitt (15) und einem zu dem ersten Faltabschnitt (15) orthogonalen Bereich umfasst.

4. Stilleinlagenhüllkörper (1A, 1 B) gemäß einem der Ansprüche 1 bis 3, wobei der Absorber (12) an dem ersten Faltabschnitt (15) im Vergleich zu dessen Umfeld mit unterschiedlichen Eigenschaften hinsichtlich Dicke oder Grundgewicht gebildet ist, wodurch ein Unterschied bei der Steifigkeit zwischen dem ersten Faltabschnitt (15) und dessen Umfeld bereitgestellt ist, oder der erste Faltabschnitt (15) durch Prägen eines Teil des Stilleinlagenhauptkörpers (10) gebildet ist, so dass ein Unterschied bei der Steifigkeit zwischen dem ersten Faltabschnitt (15) und dem Umfang (17) bereitgestellt ist.

5. Stilleinlagenhüllkörper (1A, 1 B) gemäß einem der Ansprüche 1 bis 4, wobei das elastische Element eine Schicht ist, die aus einem Schaummaterial und einem mit einem Grundgewicht von 30 g/m² oder mehr gebildeten Vliesstoff gebildet ist.

6. Stilleinlagenhüllkörper (1A, 1 B) gemäß einem der Ansprüche 1 bis 5, wobei der erste Faltabschnitt (15) diskontinuierlich in einem vorher festgelegten Abstand bereitgestellt ist.

7. Stilleinlagenhüllkörper (1A, 1 B) gemäß einem der Ansprüche 1 bis 6, wobei der erste Faltabschnitt (15) in einer diskontinuierlichen Weise getrennten Bereichen auf beiden Seiten über einen zentralen Bereich des Stilleinlagenhauptkörpers (10) hinweg bereitgestellt ist und dem Abschnitt des Stilleinlagenhauptkörpers (10), der mit der Brustwarze der Benutzerin in Kontakt ist, wenn der Stilleinlagenhauptkörper (10) getragen wird, nicht bereitgestellt ist.

8. Stilleinlagenhüllkörper (1A, 1 B) gemäß einem der Ansprüche 1 bis 6, wobei der erste Faltabschnitt (15) nur einer Seite über einen zentralen Bereich des Stilleinlagenhauptkörpers (10) hinweg bereitgestellt ist und dem Abschnitt des Stilleinlagenhauptkörpers (10), der mit der Brustwarze der Benutzerin in Kontakt ist, wenn der Stilleinlagenhauptkörper (10) getragen wird, nicht bereitgestellt ist.

9. Stilleinlagenhüllkörper (1A, 1 B) gemäß einem der Ansprüche 1 bis 8, der ferner einen zweiten Faltabschnitt, der so bereitgestellt ist, dass er nahe dem Scheitel des Stilleinlagenhauptkörpers (10) verläuft und sich zu dessen Umfang (17) erstreckt, beinhaltet, wobei der zweite Faltabschnitt orthogonal zu dem ersten Faltabschnitt (15) bereitgestellt ist.

## Revendications

1. Un corps emballé de compresse d'allaitement (1A, 1 B) comprenant :
une compresse d'allaitement présentant une paire de corps principaux de compresse d'allaitement (10) formés selon une configuration qui permet de recouvrir les seins d'une utilisatrice ; et
un élément d'emballage (2A, 2B) qui emballe la compresse d'allaitement,
chaque corps principal de compresse d'allaitement (10) étant formé en configuration de dôme présentant une face incurvée avec un sommet prévu au niveau d'une extrémité de dessus de celui-ci, chaque corps principal de compresse d'allaitement (10) comprenant :
un feuillet imperméable à du liquide (11) prévu sur une face externe du corps principal de compresse d'allaitement (10) ;
un feuillet perméable à du liquide (13), prévu sur le côté sein dudit feuillet imperméable à du liquide (11) et joint audit feuillent imperméable à du liquide (11) au niveau de la périphérie de celui-ci ;
un absorbeur (12), prévu entre ledit feuillet imperméable à du liquide (11) et ledit feuillet perméable à du liquide (13), lequel absorbe et retient le lait maternel d'une mère ;
**caractérisé en ce que**
chaque corps principal de compresse d'allaitement (10) comprend une première portion de pliage (15), arrangée de telle sorte qu'elle passe près du sommet dudit corps principal de compresse d'allaitement (10) et s'étende jusqu'aux environs d'un périmètre (17) dudit corps principal de compresse d'allaitement (10), laquelle sert d'axe de pliage le long duquel ledit corps principal de compresse d'allaitement (10) peut être plié ; et
le feuillet imperméable à du liquide (11) présente un moyen sensible au fléchissement positionné en travers de la première portion de pliage (15), le moyen sensible au fléchissement étant un élément élastique.

2. Le corps emballé de compresse d'allaitement (1A, 1 B) selon la revendication 1, dans lequel ledit corps principal de compresse d'allaitement (10) est emballé par l'élément d'emballage (2A, 2B), dans un état plié en deux, ladite première portion de pliage (15) faisant office d'axe de pliage.

3. Le corps emballé de compresse d'allaitement (1A, 1 B) selon la revendication 1, dans lequel ledit corps principal de compresse d'allaitement (10) inclut en outre un moyen de fixation (16) destiné à fixer ledit corps principal de compresse d'allaitement (10) au vêtement de l'utilisatrice au niveau d'une région autre que ladite première portion de pliage (15) et au niveau d'une région orthogonale à ladite première portion de pliage (15).

4. Le corps emballé de compresse d'allaitement (1A, 1B) selon une quelconque des revendications 1 à 3, dans lequel l'absorbeur (12) est formé avec différentes propriétés en matière d'épaisseur ou de poids de base au niveau de la première portion de pliage (15) par comparaison aux environs de celui-ci, prévoyant dès lors une différence en matière de rigidité entre la première portion de pliage (15) et les environs de celle-ci, ou la première portion de pliage (15) est formée en gaufrant une partie du corps principal de compresse d'allaitement (10), si bien qu'une différence en matière de rigidité est prévue entre ladite première portion de pliage (15) et le périmètre (17).

5. Le corps emballé de compresse d'allaitement (1A, 1 B) selon une quelconque des revendications 1 à 4, dans lequel l'élément élastique est un feuillet formé en un matériau mousse et un tissu non tissé formé avec un poids de base de 30 g/m² ou plus.

6. Le corps emballé de compresse d'allaitement (1A, 1 B) selon une quelconque des revendications 1 à 5, dans lequel la première portion de pliage (15) est prévue de façon discontinue à une inclinaison prédéterminée.

7. Le corps emballé de compresse d'allaitement (1A, 1 B) selon une quelconque des revendications 1 à 6, dans lequel la première portion de pliage (15) est prévue d'une manière discontinue pour séparer des régions sur les deux côtés en travers d'une région centrale du corps principal de compresse d'allaitement (10) et n'est pas prévue sur la portion du corps principal de compresse d'allaitement (10) qui est en contact avec le mamelon de l'utilisatrice lorsque le corps principal de compresse d'allaitement (10) est porté.

8. Le corps emballé de compresse d'allaitement (1A, 1 B) selon une quelconque des revendications 1 à 6, dans lequel la première portion de pliage (15) est prévue sur un côté seulement en travers d'une région centrale du corps principal de compresse d'allaitement (10) et n'est pas prévue sur la portion du corps principal de compresse d'allaitement (10) qui est en contact avec le mamelon de l'utilisatrice lorsque le corps principal de compresse d'allaitement (10) est porté.

9. Le corps emballé de compresse d'allaitement (1A, 1 B) selon une quelconque des revendications 1 à 8, comprenant en outre une deuxième portion de pliage prévue de telle sorte qu'elle passe près du sommet du corps principal de compresse d'allaitement (10) et s'étende jusqu'au périmètre (17) de celui-ci, la deuxième portion de pliage étant prévue de façon orthogonale à la première portion de pliage (15).
